# EUROPÄISCHE PATENTSCHRIFT

(11) **EP 3 689 397 B1**
(45) Veröffentlichungstag und Bekanntmachung des Hinweises auf die Patenterteilung: **23.04.2025**
(21) Anmeldenummer: 19154910.4
(22) Anmeldetag: 31.01.2019
(51) Int. Cl.: A61M 5/31, A61M 5/34

(54) **SPRITZENKÖRPER, SPRITZE UND INJEKTIONSVORRICHTUNG ZUM INJIZIEREN EINES HOCHVISKOSEN MEDIUMS**
SYRINGE BODY, SYRINGE AND INJECTION DEVICE FOR INJECTING A HIGHLY VISCOUS MEDIUM
CORPS DE SERINGUE, SERINGUE ET DISPOSITIF D'INJECTION PERMETTANT D'INJECTER UN MILIEU À HAUTE VISCOSITÉ

(43) Veröffentlichungstag der Anmeldung: 05.08.2020
(73) Patentinhaber: SCHOTT Pharma Schweiz AG, 9000 St. Gallen (CH)
(72) Erfinder: Moser, Raymond, 9032 Engelburg (CH); van Ginneken, Tom, 9404 Rorschacherberg (CH); Brechler, Sebastian, 9230 Flawil (CH)
(74) Vertreter: Fuchs Patentanwälte Partnerschaft mbB

(56) Entgegenhaltungen:
- US-A- 5 554 133
- US-A1- 2003 220 613
- US-A1- 2010 152 679
- US-A1- 2016 184 528
- SCHOTT: "TSK HPC: Advanced hub @BULLET Advanced hub geometry @BULLET ULTRA thin wall @BULLET External threading @BULLET Hard polymer", 19 December 2018 (2018-12-19), XP055585171, Retrieved from the Internet <URL:https://www.schott.com/innovation/en/wp-content/uploads/sites/2/2019/01/SCHOTT_PP_DB_TopPac_TSK_A4_RZ_2018_12_19.pdf> [retrieved on 20190502]
- "ISO 80369-7 : Small-bore connectors for liquids and gases in healthcare applications -- Part 7: Connectors for intravascular or hypodermic applications", ISO STANDARD, ISO, CH, vol. ISO-80369-7, 1 October 2016 (2016-10-01), pages 1 - 43, XP009512919

## Beschreibung

Die vorliegende Erfindung betrifft eine Injektionsvorrichtung zum Injizieren eines hochviskosen Mediums. Insbesondere kann die Injektionsvorrichtung zum Injizieren oder Applizieren von hochviskosen pharmazeutischen Medien verwendet werden.

### Hintergrund der Erfindung

Spritzen finden beispielsweise im medizinischen und kosmetischen Bereich Anwendung und dienen dazu, ein in der Spritze aufgenommenes Medium in den Körper eines Patienten zu injizieren. So werden im Rahmen einer kosmetischen Behandlung der Haut eines Patienten z.B. Dermalfüller unter die Hautoberfläche des Patienten gespritzt. Bei kosmetischen Anwendungen kommen meist hochviskose Medien zum Einsatz. In der Spritze aufgenommene Medien können verschiedene Formen von Fluiden, pastösen oder flüssigen Substanzen sowie Gemischen umfassen.

Das Dokument US 2010/152679 A1 offenbart eine Spritze, die zum Injizieren einer viskosen Flüssigkeit mit einer Nadelanordnung verbindbar ist. Um eine ausreichend starke Verbindung zwischen Spritzenkörper und Nadelanordnung zu realisieren und ein Ablösen zu verhindern, schlägt das Dokument US 2010/152679 A1 vor, ein Gewinde eines Verbindungsabschnitts zwischen der Spritze und der Nadelanordnung mit einer Steigung von 3 mm zu versehen.

Das Dokument US 2003/220613 A1 offenbart ebenfalls eine Spritze mit einem Spritzenkörper, der an einem Ende mit einer Nadelanordnung verbindbar ist. Spritzen mit einer umlaufenden Fase am proximalen Ende des Spritzenkörpers sind unter anderem bekannt aus: US5554133A sowie US2016/184528A1.

Um die für das Injizieren erforderliche Hautperforation und damit verbundene Schmerzen für den Patienten möglichst gering zu halten, werden bevorzugt Nadeln mit sehr geringer Kanülendicke ("Gauge") vorgesehen. Dies gilt insbesondere für Anwendungen im Gesicht eines Patienten.

Die Kombination aus möglichst geringer Kanülendicke und zu injizierenden Medien hoher Viskosität führt zu hohen Beanspruchungen der Spritze bzw. des Spritzenkörpers. Insbesondere können hohe Drücke innerhalb der Spritze zu einem mechanischen Versagen der Spritzenanordnung führen. Da ein mechanisches Versagen der Spritze jedoch zu vermeiden ist, sind bei bisher bekannten Spritzen die Möglichkeiten des Einsatzes von Nadeln mit geringer Kanülendicke begrenzt, was wiederrum die vorstehend beschriebenen Nachteile für den Patienten mit sich bringt.

Es ist daher eine Aufgabe der vorliegenden Erfindung, die Nachteile aus dem Stand der Technik zu überwinden. Insbesondere besteht eine Aufgabe der Erfindung darin, eine Injektionsvorrichtung bereitzustellen, die die Verwendung von Nadeln mit geringer Kanülendicke zum Injizieren von hochviskosen Medien ermöglichen.

Die Aufgaben werden durch eine Injektionsvorrichtung gemäß den unabhängigen Patentansprüchen gelöst. Weiterbildungen und Ausführungsformen der Injektionsvorrichtung sind Gegenstand der abhängigen Ansprüche und der nachstehenden Beschreibung.

### Beschreibung der Erfindung

Ein Aspekt der Erfindung betrifft eine Injektionsvorrichtung zum Injizieren bzw. Applizieren eines hochviskosen Mediums, umfassend eine Spritze mit einem Spritzenkörper und eine Nadelanordnung. In einer Ausführungsform ist das hochviskose Medium charakterisiert durch einen Speichermodul G' von wenigstens 30 Pa und höchstens 150 Pa, insbesondere wenigstens 50 Pa und höchstens 100 Pa, oder wenigstens 70 und höchstens 90 Pa. Der Verlustfaktor tan δ kann zwischen 0,2 und 0,8, insbesondere zwischen 0,3 und 0,6 oder zwischen 0,4 und 0,5 betragen. Dies können z.B. Hyaluronsäure-Filler sein. Die Viskosität kann mit einem Platte-Platte-Messsystem bei 25°C und einem Luftdruck von 1013,25 hPa gemessen werden (z.B. Rheometer Firma: Anton Paar MCR 302), insbesondere mit einer Frequenz von 1 Hz. Die Messung kann beispielsweise mit dem Verfahren nach ISO Norm 6721-10-2015-09 erfolgen. Der Spritzenkörper umfasst einen distalen Endabschnitt und einen proximalen Endabschnitt. Der Spritzenkörper ist hohlzylindrisch und bildet eine Kammer zum Aufnehmen des hochviskosen Mediums. Der proximale Endabschnitt weist eine Öffnung auf, durch die eine Kolbenstangenanordnung in die Kammer einführbar oder eingeführt ist. Die Kolbenstangenanordnung ist in der Kammer in Richtung einer Kammerlängsachse verlagerbar und gleitend in dem Spritzenkörper geführt.

An dem distalen Endabschnitt des Spritzenkörpers ist ein Luer-Lock-Verbinder ausgebildet, der einen Außenkegel mit einer weiteren Öffnung zum Ausgeben des hochviskosen Mediums und einen hülsenförmigen Abschnitt mit einem Innengewinde umfasst. Der Luer-Lock-Verbinder bildet einen Teil eines Luer-Lock-Verbindungssystems und ist dazu ausgebildet, mit einem an einer Nadelanordnung ausgebildeten Luer-Lock-Verbinder-Gegenstück zusammenzuwirken. Der Außenkegel kann auch als eine über ein distales Ende des Spritzenkörpers hinausstehende konische Düse beschrieben werden, durch die das hochviskose Medium aus der Kammer ausführbar ist. Der Außenkegel stellt ebenfalls einen Teil des Luer-Lock-Verbindungssystems dar und wirkt ebenfalls mit einem komplementären Gegenstück der Nadelanordnung zusammen. In einem Luer-Lock-Verbindungssystem sind der Außenkegel und der diesen umgebende hülsenförmige Abschnitt koaxial zueinander angeordnet. Der Luer-Lock-Verbinder des Spritzenkörpers kann insbesondere im Rahmen von ISO Norm 80369-7:2016-12-01 ausgebildet und dimensioniert sein. Dies ermöglicht eine Verwendung des erfindungsgemäßen Spritzenkörpers mit einer Vielzahl an verschiedenen, im Bereich der Luer-Lock-Verbindung genormten Nadelanordnungen.

Der Spritzenkörper hat erfindungsgemäß eine NPO-Mindestwiderstandsfähigkeit wischen 90 N und 105 N, vorzugsweise zwischen 95 N und 103,5 N, bevorzugt zwischen 98 N und 102 N. NPO steht für "Needle Pop Off" und bezeichnet das schlagartige Lösen bzw. Abspringen einer Nadelanordnung von einem Spritzenkörper, insbesondere einer über eine Luer-Lock-Verbindung mit dem Spritzenkörper verbundenen Nadelanordnung. Unter hohem Druck (ausgelöst durch Kraftaufbringung auf den Spritzenkörper über die Kolbenstangenanordnung) beginnt die Nadelanordnung sich zu drehen und sich mit hoher Geschwindigkeit von dem Spritzenkörper zu lösen, d.h. ein zu dem Außenkegel des Spritzenkörpers komplementärer Innenkegel der Nadelanordnung löst sich.

Die NPO-Mindestwiderstandsfähigkeit ("Needle Pop Off"-Mindestwiderstandsfähigkeit) gibt einen Schwellenwert einer Kraft an, die im Rahmen des nachstehend beschriebenen Testverfahrens auf eine in den Spritzenkörper eingebrachte und mit diesem in Wirkverbindung stehende Kolbenstangenanordnung aufgebracht wird. Die NPO-Mindestwiderstandsfähigkeit ist dadurch definiert, dass unterhalb dieses Schwellenwerts bei nicht mehr als 1,8% der getesteten Spritzenkörper ein "Needle Pop Off" auftritt. Es sind mindestens 56 Messungen von Spritzenkörpern derselben Konfiguration und Ausführung erforderlich, um ein aussagekräftiges Ergebnis für die NPO-Mindestwiderstandsfähigkeit zu erhalten.

Das Testverfahren zum Messen der NPO-Widerstandsfähigkeit, insbesondere der NPO-Mindestwiderstandsfähigkeit und der durchschnittlichen NPO-Widerstandsfähigkeit, sieht vor, dass der zu testende Spritzenkörper senkrecht in einer Prüfmaschine angeordnet und im Bereich des proximalen Endes des Spritzenkörpers gehalten wird. Die verwendete Prüfmaschine ist eine Universalprüfmaschine der Firma "TesT", Model "TesT 106.2 kN". Für das Testverfahren ist diese oder eine damit vergleichbare Universalprüfmaschine zu verwenden.

Der zu testende Spritzenkörper ist über den Luer-Lock-Verbinder mit einer Nadelanordnung verbunden, die zum Herstellen der Luer-Lock-Verbindung mittels eines Luer-Lock-Verbinder-Gegenstücks mit einem Drehmoment von 12 Ncm auf den distalen Endabschnitt des Spritzenkörpers aufgeschraubt ist. Konkret soll das beschriebene Testverfahren mit der Nadelanordnung "TSK STERiJECT Hypodermic Needle", Ref.: PRC-30013I, 30 G x ½ oder einer damit vergleichbaren Nadelanordnung durchgeführt werden. Die für das Testverfahren verwendete Nadelanordnung umfasst als Luer-Lock-Verbinder-Gegenstück einen Innenkegel und zwei an einem Außenumfang eines Nadelansatzes der Nadelanordnung angeordnete Finnen. Die für das Testverfahren verwendete Nadelanordnung umfasst eine Kanüle bzw. Hohlnadel mit einer Dicke von 30 G und einer Länge von 13 mm (30 G x ½). Die Kanüle ist zu Testzwecken vor Durchführung des Testverfahrens mittels eines Hammers zu plätten und dadurch zu verschließen. Für das Testverfahren ist eine trockene Luer-Lock-Verbindung herzustellen, indem die Nadelanordnung auf den Spritzenkörper aufgeschraubt wird, bevor anschließend ein hochviskoses Medium in die Kammer gefüllt wird. Das Testverfahren ist mit nicht dampfsterilisierten Komponenten (Spritzenkörper, Nadelanordnung, Kolbenstangenanordnung) durchzuführen.

Für das vorliegende Testverfahren ist ein hochviskoses Medium mit einem Speichermodul G' von etwa 84,5 Pa und einem Verlustfaktor tan δ von etwa 0,48 zu wählen. Die Viskosität kann mit einem Platte-Platte-Messsystem bei 25°C und einem Luftdruck von 1013,25 hPa gemessen werden (z.B. Rheometer Firma: Anton Paar MCR 302), insbesondere mit einer Frequenz von 1 Hz. Die Messung kann beispielsweise mit dem Verfahren nach ISO Norm 6721-10-2015-09 erfolgen. Der zu testende Spritzenkörper ist bestimmungsgemäß vollständig mit dem hochviskosen Material gefüllt. Ferner ist eine Kolbenstangenanordnung zu Beginn des Testverfahrens in den Spritzenkörper eingebracht und steht mit diesem in Wirkverbindung, jedoch befinden sich Spritzenkörper und Kolbenstangenanordnung zu Beginn des Testverfahrens noch in einer Ausgangsstellung, d.h. in einer Nichtbetätigungsstellung. Für das vorliegende Testverfahren ist eine für den jeweiligen zu testenden Spritzenkörper vorgesehene Standardkolbenstangenanordnung zu verwenden. Beispielsweise kann bei einem Spritzenkörper mit 5 mm Innendurchmesser der Kammer ein Standardkolben der Art FM257 verwendet werden (z.B. des Herstellers Daetwyler).

Über einen Prüfstempel der Prüfmaschine wird bei dem Testverfahren eine Kraft senkrecht auf ein proximales Ende der Kolbenstangenanordnung aufgebracht. Der Prüfstempel bewegt sich mit einer konstanten Prüfgeschwindigkeit von 12,6 mm/min in Richtung des distalen Endabschnitts des Spritzenkörpers, was die auf die Kolbenstangenanordnung einwirkende Kraft kontinuierlich bis maximal 420 N erhöht. Der Prüfstempel wird beim Testen bis zu einem Weg von 15 mm verlagert. Die einwirkende Kraft wird mittels eines Kraftsensors mit einer Abtastrate von 200 Hz detektiert. Der Prüfstempel wird solange weiterbewegt bzw. wird die einwirkende Kraft solange erhöht, bis Undichtigkeit ("*Leakage*") und/oder NPO auftritt oder bis die Maximalkraft von 420 N erreicht ist. Der Test wird gestoppt, d.h. Undichtigkeit ("*Leakage*") und/oder NPO wird erkannt, wenn die gemessene Kraft plötzlich um wenigstens 30 % abfällt. Die einwirkende Kraft zum Zeitpunkt des Auftretens von Undichtigkeit und/oder NPO wird dokumentiert und mit der Information verknüpft, ob Undichtigkeit und/oder NPO bei dieser Kraft aufgetreten ist. Aus den dokumentierten Messergebnissen lassen sich dann die oben beschriebene NPO-Mindestwiderstandsfähigkeit und die durchschnittliche NPO-Widerstandsfähigkeit bestimmen. Die hier untersuchte Undichtigkeit ("*Leakage*") ist die durch die aufgebrachte Kraft und des dadurch wirkenden Drucks auftretende Undichtigkeit zwischen dem Spritzenkörper und der Nadelanordnung. Mit anderen Worten kann die Kraft bestimmt werden, bei der zwischen dem Spritzenkörper und der Nadelanordnung im Bereich der Luer-Lock-Verbindung das hochviskose Medium ungewollt austritt.

Ein zu vermeidendes mechanisches Versagen kann insbesondere durch NPO, durch Undichtigkeit ("*Leakage*") zwischen miteinander verbundenen Spritzenkomponenten und/oder durch einen Bruch des Spritzenkörpers auftreten. Die Erfinder der vorliegenden Erfindung haben erkannt, dass für eine Verwendung von hochviskosen Medien und einer geringen Kanülendicke von mindestens 31 G ein Spritzenkörper vorteilhaft ist, der die vorstehend definierte NPO-Mindestwiderstandsfähigkeit hat. Ein kleinerer Nadel- bzw. Kanülendurchmesser (bspw. über 30 G) bedeutet weniger Schmerzen für den Patienten als größere Nadel- bzw. Kanülendurchmesser (bspw. herkömmlich mit hochviskosen Medien verwendete Durchmesser von 27 G), erfordert jedoch gleichzeitig eine höhere Kraftaufbringung als ein größerer Nadel- bzw. Kanülendurchmesser, um das hochviskose Medium auszugeben. Die vorstehenden erfindungsgemäßen Werte für die NPO-Mindestwiderstandsfähigkeit berücksichtigen die Erkenntnis, dass männliche Anwender bei bestimmungsgemäßer Verwendung eine Spritze bzw. Injektionsvorrichtung mit einer Maximalkraft (maximalen Fingerkraft) von durchschnittlich 95 N betätigen können. Weibliche Anwender können bei bestimmungsgemäßer Verwendung eine Spritze bzw. Injektionsvorrichtung mit einer Maximalkraft (maximalen Fingerkraft) von durchschnittlich 64 N betätigen. Die vorstehend definierte NPO-Mindestwiderstandsfähigkeit stellt somit eine optimale Auslegung der Spritzenkörperstruktur dar, die ein sicheres Injizieren eines hochviskosen Mediums mit einer sehr dünnen Kanüle sicherstellt und dennoch nicht überdimensioniert ist.

In einer Weiterbildung kann der Spritzenkörper eine durchschnittliche NPO-Widerstandsfähigkeit von wenigstens 100 N, vorzugsweise wenigstens 105 N, bevorzugt wenigstens 110 N, weiter bevorzugt wenigstens 115 N, noch weiter bevorzugt wenigstens 117 N haben. Insbesondere kann die durchschnittliche NPO-Widerstandsfähigkeit zwischen 100 N und 120 N, vorzugsweise zwischen 105 N und 120 N, bevorzugt zwischen 110 N und 120 N, weiter bevorzugt zwischen 115 N und 117 N liegen.

Die durchschnittliche NPO-Widerstandsfähigkeit gibt den Mittelwert der Kraft an, die im Rahmen des vorstehend beschriebenen Testverfahrens auf eine in den Spritzenkörper eingebrachte und mit diesem in Wirkverbindung stehende Kolbenstangenanordnung aufgebracht werden muss, damit ein Needle Pop Off auftritt. Auch hierfür sind mindestens 56 Messungen von Spritzenkörpern derselben Konfiguration und Ausführung erforderlich, um ein aussagekräftiges Ergebnis für die durchschnittliche NPO-Widerstandsfähigkeit zu erhalten.

In einer Ausführungsform kann der Spritzenkörper eine Leakage-Mindestwiderstandsfähigkeit von wenigstens 100 N haben. Vorzugsweise kann der Spritzenkörper eine Leakage-Mindestwiderstandsfähigkeit von über 105 N, bevorzugt über 117,5 N, weiter bevorzugt über 125 N haben. Insbesondere kann der Spritzenkörper eine Leakage-Mindestwiderstandsfähigkeit zwischen 100 N und 130 N haben, vorzugsweise zwischen 105 N und 120 N, bevorzugt zwischen 110 N und 115 N.

In einer Weiterbildung kann der Spritzenkörper eine durchschnittliche Leakage-Widerstandsfähigkeit von wenigstens 115 N, vorzugsweise wenigstens 120 N, bevorzugt wenigstens 123 N, weiter bevorzugt wenigstens 125 N, noch weiter bevorzugt wenigstens 127 N haben. Insbesondere kann die durchschnittliche Leakage-Widerstandsfähigkeit zwischen 110 N und 135 N, vorzugsweise zwischen 115 N und 130 N, bevorzugt zwischen 120 N und 130 N, weiter bevorzugt zwischen 125 N und 130 N liegen.

Die Leakage-Mindestwiderstandsfähigkeit (Undichtigkeits-Mindestwiderstandsfähigkeit) gibt einen Schwellenwert einer Kraft an, die im Rahmen des vorstehend beschriebenen Testverfahrens auf eine in den Spritzenkörper eingebrachte und mit diesem in Wirkverbindung stehende Kolbenstangenanordnung aufgebracht wird. Die Leakage-Mindestwiderstandsfähigkeit ist dadurch definiert, dass unterhalb dieses Schwellenwerts bei weniger als 1,8% der getesteten Spritzenkörper eine Undichtigkeit auftritt. Es sind mindestens 56 Messungen von Spritzenkörpern derselben Konfiguration und Ausführung erforderlich, um ein aussagekräftiges Ergebnis für die Leakage-Mindestwiderstandsfähigkeit zu erhalten.

Ferner gibt die durchschnittliche Leakage-Widerstandsfähigkeit den Mittelwert der Kraft an, die im Rahmen des vorstehend beschriebenen Testverfahrens auf eine in den Spritzenkörper eingebrachte und mit diesem in Wirkverbindung stehende Kolbenstangenanordnung aufgebracht werden muss, damit eine Undichtigkeit auftritt. Auch hierfür sind mindestens 56 Messungen von Spritzenkörpern derselben Konfiguration und Ausführung erforderlich, um ein aussagekräftiges Ergebnis für die durchschnittliche Leakage-Widerstandsfähigkeit zu erhalten.

In einer Ausführungsform kann das Innengewinde des Luer-Lock-Verbinders einen Innendurchmesser von maximal 7,15 mm oder maximal 7,12 mm haben. Der Innendurchmesser kann vorzugsweise wenigstens 7,05 mm oder wenigstens 7,08 mm betragen. In bevorzugten Ausführungsformen liegt der Innendurchmesser zwischen 7,05 mm und 7,15 mm, vorzugsweise zwischen 7,08 mm und 7,12 mm, bevorzugt liegt er bei maximal 7,1 mm. Der Innendurchmesser beschreibt den kleinsten Innendurchmesser des hülsenförmigen Endabschnitts, d.h. gemessen von Gewindekamm zu gegenüberliegendem Gewindekamm des Innengewindes. Ein Innendurchmesser mit dieser Abmessung kann sich positiv auf eine ausreichend enge Verbindung zwischen dem Spritzenkörper und einer damit verbundenen Nadelanordnung auswirken und so das Einstellen der NPO-Mindestwiderstandsfähigkeit sowie der durchschnittlichen NPO-Widerstandsfähigkeit vorteilhaft beeinflussen. Analog gilt dies in Bezug auf die Leakage-Mindestwiderstandsfähigkeit und die durchschnittliche Leakage-Widerstandsfähigkeit.

Gemäß einer Ausführungsform des Spritzenkörpers kann das Innengewinde an dem Kamm des Gewindeprofils eine Breite von mindestens 0,44 mm oder mindestens 0,46 mm aufweisen. Bevorzugt beträgt die Breite maximal 0,52 mm oder maximal 0,50 mm. In bevorzugten Ausführungsformen kann die Breite zwischen 0,44 mm und 0,52 mm, vorzugsweise zwischen 0,46 mm und 0,50 mm, bevorzugt 0,48 mm betragen.

Zusätzlich oder alternativ dazu kann das Innengewinde an dem Grund des Gewindeprofils eine Breite von mindestens 0,85 mm oder mindestens 0,875 mm aufweisen. Bevorzugt beträgt die Breite maximal 0,95 mm oder maximal 0,925 mm. In bevorzugten Ausführungsformen kann die Breite zwischen 0,85 mm und 0,95 mm, vorzugsweise zwischen 0,875 mm und 0,925 mm, bevorzugt 0,9 mm betragen.

Das Innengewinde kann an dem Gewindeprofil beidseitig einen Winkel zwischen 22,5° und 27,5°, insbesondere einen Winkel von 24° bis 26° oder von etwa 25° aufweisen. Der Winkel kann als der zwischen einer orthogonal zu der Längsachse des Spritzenkörpers stehenden, das Gewindeprofil schneidenden Linie und der Flanke des Gewindeprofils eingeschlossene Winkel beschrieben werden.

Durch das Ausbilden der Gewindeprofilparameter nach Maßgabe der vorstehend beschriebenen Dimensionen kann die Kontaktfläche der Gewindeverbindung, d.h. die Kontaktfläche zwischen dem Innengewinde des Spritzenkörpers und einem komplementären Außengewinde einer zugehörigen Nadelanordnung angepasst werden. Dies kann die Luer-Lock-Verbindung mit der zugehörigen Nadelanordnung verbessern und das Einstellen der NPO-Mindestwiderstandsfähigkeit sowie der durchschnittlichen NPO-Widerstandsfähigkeit vorteilhaft beeinflussen. Analog gilt dies in Bezug auf die Leakage-Mindestwiderstandsfähigkeit und die durchschnittliche Leakage-Widerstandsfähigkeit.

In einer Weiterbildung kann eine den Außenkegel abschließende mit der weiteren Öffnung versehene distale Stirnfläche des Außenkegels um einen Abstand von wenigstens 2,1 mm oder wenigstens 2,2 mm über einen distalen Kragen des hülsenförmigen Abschnitts hinausstehen. In einer Ausführungsform beträgt dieser Abstand maximal 2,5 mm oder maximal 2,4 mm. In einer bevorzugten Ausführungsform steht die distale Stirnfläche des Außenkegels um einen Abstand von 2,1 mm bis 2,5 mm, vorzugsweise von 2,2 mm bis 2,4 mm, bevorzugt von 2,3 mm über einen distalen Kragen des hülsenförmigen Abschnitts hinaus. Auch diese strukturelle Anpassung kann das Einstellen der NPO-Mindestwiderstandsfähigkeit sowie der durchschnittlichen NPO-Widerstandsfähigkeit vorteilhaft beeinflussen. Analog gilt dies in Bezug auf die Leakage-Mindestwiderstandsfähigkeit und die durchschnittliche Leakage-Widerstandsfähigkeit.

Erfindungsgemäß umfasst der Spritzenkörper an dem proximalen Endabschnitt eine Innenumfangsfläche mit einer Fase, die die Innenumfangsfläche von dem proximalen Endabschnitt in Richtung des distalen Endabschnitts betrachtet verjüngt. Mit anderen Worten ist die Innenumfangsfläche im Bereich der Fase in Richtung des distalen Endabschnitts betrachtet konisch zulaufend. Die Fase kann das Einführen der Kolbenstangenanordnung, genauer gesagt des Kolbens, in die Kammer des Spritzenkörpers vereinfachen. Die Fase hat in Richtung der Längsachse des Spritzenkörpers betrachtet eine Länge zwischen 1,2 mm und 1,8 mm, vorzugsweise von 1,4 mm bis 1,6 mm, bevorzugt von etwa 1,5 mm. Zusätzlich dazu schließt die Fase einen Winkel von wenigstens 13°, vorzugsweise von wenigstens 14°, bevorzugt von wenigstens 15° mit der Längsachse ein. Durch das Ausbilden einer derart dimensionierten Fase kann die Gesamtlänge des Spritzenkörpers gegenüber bekannten Spritzenkörpern verringert werden. Dies kann sich vorteilhaft auf die Ergonomie des Spritzenkörpers bzw. die Handhabung durch den Anwender auswirken. Dennoch kann mit einer Fase dieser Dimensionierung eine ausreichende Stabilität und Dichtigkeit (die sogenannte Container Closure Integrity, CCI) der Spritze gewährleistet werden. So kann beispielsweise vorgesehen sein, dass bei einer vollständig befüllten und nicht betätigten Spritze ein Abstand von wenigstens 9 mm bis maximal 10 mm, oder etwa 9,5 mm zwischen dem proximalen Spritzenende und einem proximalen Ende des Kolbens der Kolbenstangenanordnung vorgesehen sein.

Der Spritzenkörper kann in einer Ausführungsform eine maximale Gesamtlänge von 80 mm und einen Kammerinnendurchmesser von maximal 5 mm haben, wobei in dem Spritzenkörper ein Volumen von wenigstens 1 ml aufnehmbar ist. Genauer gesagt ist in der Kammer und einem sich daran anschließenden durch den Außenkegel gebildeten Kammerabschnitt zusammen ein Volumen von wenigstens 1 ml aufnehmbar. Die Gesamtlänge kann von einer den proximalen Endabschnitt begrenzenden Stirnfläche des Spritzenkörpers bis zu einer den distalen Endabschnitt begrenzenden Stirnfläche des Spritzenkörpers, d.h. der Stirnfläche des Außenkegels, gemessen werden. Der Kammerinnendurchmesser kann über die gesamte Länge der Kammer hinweg konstant sein. Beispielsweise kann in der Kammer und dem Kammerabschnitt gemeinsam ein Volumen von 1,073 ml aufnehmbar sein, um ein Überfüllen von 2% und ein Einschließen einer Luftblase von 1 mm Länge in der Kammer zuzulassen. Es ist anzumerken, dass das aufnehmbare Volumen nicht mit dem durch die gesamte Kammer und den Kammerabschnitt gebildeten Gesamtvolumen gleichzusetzen ist. Zusätzlich zu dem aufzunehmenden Volumen des Mediums samt vorstehend beschriebenen Toleranzen für das Überfüllen und die Luftblase, muss die Kammer bereits in einer Nichtbetätigungsstellung zusätzlich noch einen Teil der Kolbenstangenanordnung aufnehmen können und diese stabil lagern. Hierzu muss der Kolben der Kolbenstangenanordnung in einer Nichtbetätigungsstellung bereits ausreichend weit von dem proximalen Ende des Spritzenkörpers entfernt in dem Spritzenkörper aufgenommen sein. Beispielsweise kann ein Abstand von 9,5 mm zwischen dem proximalen Spritzenende und einem proximalen Ende des Kolbens der Kolbenstangenanordnung vorgesehen sein.

Der Spritzenkörper kann vorzugsweise innerhalb und gemäß ISO Norm 11040-6:2012-04-01 ausgebildet und dimensioniert sein.

Der Spritzenkörper kann in einer weiteren Ausführungsform eine maximale Gesamtlänge von 60 mm und einen Kammerinnendurchmesser von maximal 4,65 mm haben, wobei in der Kammer und dem Kammerabschnitt zusammen ein Volumen von wenigstens 0,5 ml aufnehmbar ist.

Der Spritzenkörper kann in einer weiteren Ausführungsform eine maximale Gesamtlänge von 71 mm und einen Kammerinnendurchmesser von maximal 5 mm haben, wobei in der Kammer und dem Kammerabschnitt zusammen ein Volumen von wenigstens 0,8 ml aufnehmbar ist.

Der Spritzenkörper kann in einer weiteren Ausführungsform eine maximale Gesamtlänge von 95 mm und einen Kammerinnendurchmesser von maximal 6,45 mm haben, wobei in der Kammer und dem Kammerabschnitt zusammen ein Volumen von wenigstens 2,25 ml aufnehmbar ist.

Der Spritzenkörper kann in einer weiteren Ausführungsform eine maximale Gesamtlänge von 117 mm und einen Kammerinnendurchmesser von maximal 6,45 mm haben, wobei in der Kammer und dem Kammerabschnitt zusammen ein Volumen von wenigstens 2,8 ml aufnehmbar ist.

Der Spritzenkörper kann in einer weiteren Ausführungsform eine maximale Gesamtlänge von 56 mm und einen Kammerinnendurchmesser von maximal 12,2 mm haben, wobei in der Kammer und dem Kammerabschnitt zusammen ein Volumen von wenigstens 2,8 ml aufnehmbar ist.

Der Spritzenkörper kann in einer weiteren Ausführungsform eine maximale Gesamtlänge von 58 mm und einen Kammerinnendurchmesser von maximal 12,2 mm haben, wobei in der Kammer und dem Kammerabschnitt zusammen ein Volumen von wenigstens 3,0 ml aufnehmbar ist.

Der Spritzenkörper kann in einer weiteren Ausführungsform eine maximale Gesamtlänge von 115 mm und einen Kammerinnendurchmesser von maximal 8,75 mm haben, wobei in der Kammer und dem Kammerabschnitt zusammen ein Volumen von wenigstens 5 ml aufnehmbar ist.

Die vorstehend beschriebenen Werte können Maximalmaße oder genaue Maße sein. Ein geringer Kammerinnendurchmesser wirkt sich positiv auf das Applizieren einer hochviskosen Flüssigkeit durch Aufbringen von Fingerkraft auf die Spritze bzw. die Injektionsvorrichtung aus. Gleichzeitig ist jedoch darauf zu achten, dass die Gesamtlänge des Spritzenkörpers, von der auch die Gesamtlänge der damit verwendeten Kolbenstangenanordnung abhängt, nicht zu groß gewählt wird, um die Handhabbarkeit zu gewährleisten. Die vorstehend beschriebenen Ausführungsformen stellen beispielhafte Ausführungsformen dar, die für unterschiedliche Spritzenkörpervolumina vorteilhafte Kombinationen aus Gesamtlänge und Innendurchmesser angeben.

Gemäß einer Weiterbildung kann der Spritzenkörper zumindest im Bereich der Kammer eine Wandstärke von wenigstens 1,7 mm, vorzugsweise wenigstens 1,8 mm, bevorzugt wenigstens 2,0 mm, weiter bevorzugt wenigstens 2,2 mm aufweisen. Das Ausbilden einer solchen Wandstärke kann die Formstabilität des Spritzenkörpers während der Verwendung positiv beeinflussen und dadurch mechanisches Versagen des Spritzenkörpers reduzieren. Die angegebenen Werte stellen eine optimierte Geometrie hinsichtlich des Verhältnisses zwischen Innen- und Außendurchmesser dar.

Der Spritzenkörper kann beispielsweise im Bereich der Kammer einen Außendurchmesser zwischen 5 mm und 15 mm, insbesondere zwischen 7,5 mm und 12,5 mm, oder zwischen 8,4 mm und 10 mm, insbesondere von etwa 9,4 mm aufweisen.

Der Spritzenkörper kann derart ausgebildet sein, dass er eine Extrusionskraft von weniger als 40 N aufweist, wenn ein Standardkolben mit 10 mm/min in der Kammer in Richtung des distalen Endabschnitts verlagert wird. Der Spritzenkörper kann derart ausgebildet sein, dass er eine Extrusionskraft von weniger als 75 N aufweist, wenn ein Standardkolben mit 50 mm/min in der Kammer in Richtung des distalen Endabschnitts verlagert wird. Der Spritzenkörper kann derart ausgebildet sein, dass er eine Extrusionskraft von weniger als 100 N aufweist, wenn ein Standardkolben mit 100 mm/min in der Kammer in Richtung des distalen Endabschnitts verlagert wird. Die vorstehenden Werte beziehen sich auf das Ausgeben eines hochviskosen Mediums mit einem Speichermodul G' von etwa 84,5 Pa und einem Verlustfaktor tan δ von etwa 0,48. Die Viskosität kann mit einem Platte-Platte-Messsystem bei 25°C und einem Luftdruck von 1013,25 hPa gemessen werden (z.B. Rheometer Firma: Anton Paar MCR 302), insbesondere mit einer Frequenz von 1 Hz. Die Messung kann beispielsweise mit dem Verfahren nach ISO Norm 6721-10-2015-09 erfolgen. Die Extrusionskraft beschreibt die Kraft, mit der das Medium aus einer Kanüle einer Nadelanordnung austritt, die mit dem Spritzenkörper verbunden ist. Hochviskose Medien erfordern in der Regel höhere Extrusionskräfte als niedrigviskose Medien. Die vorstehenden Werte gelten für eine Testanordnung mit einer Nadelanordnung, die eine Kanüle mit einer Dicke von 30 G und einer Länge von 13 mm umfasst, also einer Nadelanordnung 30 G x ½, insbesondere der Nadelanordnung "TSK HYPODERMIC NEEDLE", Ref.: HPC-30013I-320, 30 G x ½. Die Kanüle ist bei diesem Testverfahren offen, sodass das hochviskose Medium aus der Kanüle austreten kann. Die Nadelanordnung umfasst als Luer-Lock-Verbinder-Gegenstück ein Gewinde (im Gegensatz zu der Nadelanordnung mit Finnen). Ein Spritzenkörper mit solchen vergleichsweise geringen Extrusionskräften für hochviskose Medien erfordert weniger Kraftaufbringung durch den Anwender und ermöglicht eine verbesserte Dosierungsgenauigkeit. Somit erlauben solche Spritzenkörper trotz der Verwendung hochviskoser Medien eine Nutzung von Nadelanordnungen mit Kanülen geringer Dicke, d.h. einer Dicke von 30 G oder mehr.

In einer Ausführungsform kann der Spritzenkörper aus einem Kunststoffmaterial hergestellt sein, das vorzugsweise einen E-Modul zwischen 2800 MPa und 3300 MPa hat, insbesondere zwischen 2900 MPa und 3200 MPa (1 mm/min, ISO 527 Teil 1 und 2). Das Kunststoffmaterial kann eine Zugfestigkeit (5 mm/min, ISO 527 Teil 1 und 2) zwischen 58 MPa und 65 MPa haben, insbesondere im Bereich von 60 MPa bis 63 MPa. Das Kunststoffmaterial kann eine Wasseraufnahme von weniger als 0,01 % aufweisen (ISO 62). Ferner kann das Kunststoffmaterial eine Kugeldruckhärte zwischen 180 und 195 N/mm² haben (30-Sek.-Wert bei einer Last von 961 N gemäß ISO 2039 Teil 1). Das Kunststoffmaterial kann eine Wärmeformbeständigkeitstemperatur zwischen 120 und 180 °C haben (HDT/B 0,45 MPa, ISO 75 Teil 1 und 2). Der lineare Wärmeausdehnungskoeffizient des Kunststoffmaterials kann 6,0 × 10⁻⁴ K⁻¹ betragen (ISO 11 359 Teil 1 und 2). Die Bruchdehnung des Kunststoffmaterials kann im Bereich von 2,5 bis 2,7 % liegen (ISO 527 Teil 1 und 2. Die Schlagzähigkeit des Kunststoffmaterials kann etwa 15 kJ/m² betragen (ISO 179/1eU) und/oder die Kerbschlagzähigkeit kann im Bereich von 1,6 kJ/m² bis 1,8 kJ/m² liegen (ISO 179/1eA).

Der Spritzenkörper kann in einer Weiterbildung an seinem proximalen Endabschnitt einen Flansch umfassen. Der Flansch kann einen Griff zum Abstützen von Zeigefinder und Mittelfinger eines Anwenders bei der Verwendung des Spritzenkörpers bilden oder mit einem solchen Griff versehen sein.

Die Spritze zum Injizieren eines hochviskosen Mediums mit einem Spritzenkörper der vorstehend beschriebenen Art umfasst eine Kolbenstangenanordnung. Die Kolbenstangenanordnung umfasst eine Kolbenstange und einen an ein distales Ende der Kolbenstange angebrachten Kolben. Der Kolben ist über die Öffnung an dem proximalen Endabschnitt des Spritzenkörpers in der Kammer aufgenommen und in dieser verlagerbar geführt. Der Kolben kann beispielsweise drei Dichtlippen umfassen.

Die Spritze kann ein in der Kammer des Spritzenkörpers aufgenommenes hochviskoses Medium umfassen. In einer Ausführungsform ist das hochviskose Medium charakterisiert durch einen Speichermodul G' von wenigstens 30 Pa und höchstens 150 Pa, insbesondere wenigstens 50 Pa und höchstens 100 Pa, oder wenigstens 70 und höchstens 90 Pa. Der Verlustfaktor tan δ kann zwischen 0,2 und 0,8, insbesondere zwischen 0,3 und 0,6 oder zwischen 0,4 und 0,5 betragen. Die Viskosität kann mit einem Platte-Platte-Messsystem bei 25°C und einem Luftdruck von 1013,25 hPa gemessen werden (z.B. Rheometer Firma: Anton Paar MCR 302), insbesondere mit einer Frequenz von 1 Hz. Die Messung kann beispielsweise mit dem Verfahren nach ISO Norm 6721-10-2015-09 erfolgen. Beispielsweise kann die Spritze in der Kammer des Spritzenkörpers aufgenommene Hyaluronsäure-Filler und/oder andere kosmetische Zusammensetzungen umfassen.

Die Spritze kann eine Greiflänge zwischen 6 cm und 12 cm, insbesondere zwischen 7,5 cm und 8 cm haben. Die Greiflänge bezeichnet die Länge zwischen dem proximalen Ende des Spritzenkörpers und einem proximalen Ende der Kolbenstangenanordnung. Die Kolbenstangenanordnung kann an dem proximalen Ende mit einem Griff versehen sein.

Die Nadelanordnung umfasst einen Nadelansatz und eine Kanüle, wobei der Nadelansatz zum Herstellen der Luer-Lock-Verbindung zwischen dem Spritzenkörper und der Nadelanordnung ein zu dem Luer-Lock-Verbinder des Spritzenkörpers komplementäres Luer-Lock-Verbinder-Gegenstück umfasst. Demnach umfasst der Nadelansatz einen zu dem Außenkegel des Spritzenkörpers komplementären Innenkegel und vorzugsweise ein Außengewinde, das komplementär zu dem Innengewinde des Spritzenkörpers ist. Alternativ zu einem Außengewinde kann der Nadelansatz zwei an dem Außenumfang des Nadelansatzes angeordnete Finnen umfassen.

Die Nadelanordnung kann eine Kanüle mit einer Dicke von mindestens 30 G (Gauge), vorzugsweise mindestens 31 G, bevorzugt mindestens 32 G, bevorzugt von mindestens 33 G, weiter bevorzugt von mindestens 34 G, noch weiter bevorzugt von mindestens 35 G umfassen. Die Kanüle kann eine Länge von wenigstens 10 mm, vorzugsweise wenigstens 11 mm, bevorzugt wenigstens 12 mm, weiter bevorzugt wenigstens 13 mm haben. Alternativ kann die Kanüle eine Länge von 25 mm oder mehr haben.

Der Nadelansatz der Nadelanordnung kann einen Innenkegel mit einer Länge entlang einer Längsachse der Nadelanordnung zwischen 3 mm und 7 mm umfassen, vorzugsweise zwischen 5,5 mm und 6,5 mm, bevorzugt von etwa 6.1 mm.

Insbesondere kann die Injektionsvorrichtung die Nadelanordnung "TSK HYPODERMIC NEEDLE", Ref.: HPC-30013I-320, 30 G x ½ oder eine damit vergleichbare Nadelanordnung umfassen.

Alternativ kann die Injektionsvorrichtung die Nadelanordnung "TSK STERiJECT Hypodermic Needle", Ref.: PRC-30013I, 30 G x ½ oder eine damit vergleichbare Nadelanordnung umfassen.

In einer Ausführungsform kann der Spritzenkörper aus einem Kunststoffmaterial aufgebaut sein, das ein Cycloolefin-Copolymer (COC) und/oder ein Cycloolefin-Polymer (COP) umfasst oder daraus besteht.

In einer Weiterbildung kann das Kunststoffmaterial eines oder mehrere Additive umfassen, wobei ein Additiv vorzugsweise ein Farbstoff sein kann.

Obgleich einige Aspekte und Merkmale vorstehend und nachstehend lediglich in Bezug auf den Spritzenkörper beschrieben sind, können diese Aspekte und Merkmale entsprechend für die Spritze und/oder die Injektionsvorrichtung gelten und umgekehrt.

Die Erfindung betrifft auch ein kosmetisches Verfahren, umfassend Applizieren einer hochviskosen kosmetischen Zubereitung unter Verwendung einer hierin beschriebenen Injektionsvorrichtung. In einer Ausführungsform ist die hochviskose kosmetische Zubereitung charakterisiert durch einen Speichermodul G' von wenigstens 30 Pa und höchstens 150 Pa, insbesondere wenigstens 50 Pa und höchstens 100 Pa, oder wenigstens 70 und höchstens 90 Pa. Der Verlustfaktor tan δ kann zwischen 0,2 und 0,8, insbesondere zwischen 0,3 und 0,6 oder zwischen 0,4 und 0,5 betragen. Die Viskosität kann mit einem Platte-Platte-Messsystem bei 25°C und einem Luftdruck von 1013,25 hPa gemessen werden (z.B. Rheometer Firma: Anton Paar MCR 302), insbesondere mit einer Frequenz von 1 Hz. Die Messung kann beispielsweise mit dem Verfahren nach ISO Norm 6721-10-2015-09 erfolgen. Das Applizieren kann ein Injizieren der kosmetischen Zubereitung in einen zu behandelnden Organismus umfassen. Bevorzugt wird die Zubereitung in einen Applikationsort an einem menschlichen Organismus appliziert. Die Applikation kann eine subkutane Injektion sein. Der Applikationsort kann ein menschliches Gesicht bzw. ein Abschnitt desselben sein.

### Kurzbeschreibung der Figuren

Ausführungsbeispiele der vorliegenden Erfindung werden nachstehend in Bezug auf die beiliegenden schematischen Figuren näher erläutert. Es stellen dar:
- Fig. 1: verschiedene perspektivische Ansichten einer Injektionsvorrichtung gemäß einem Ausführungsbeispiel der Erfindung.
- Fig. 2: eine Schnittdarstellung des Luer-Lock-Verbinders eines Ausführungsbeispiels eines Spritzenkörpers.
- Fig. 3: eine Detailansicht eines Gewindeprofils aus Fig. 2.
- Fig. 4: eine Schnittdarstellung eines Ausführungsbeispiels des Spritzenkörpers mit darin aufgenommener Kolbenstangenanordnung.
- Fig. 5: eine Detailansicht eines Fase des Spritzenkörpers aus Fig. 4.
- Fig. 6: ein Diagramm mit Testergebnissen eines erfindungsgemäßen Spritzenkörpers zu NPO und Leakage.
- Fig. 7: ein Diagramm mit Testergebnissen eines erfindungsgemäßen Spritzenkörpers zu Extrusionskräften.

### Figurenbeschreibung

Gleiche Bezugszeichen in den Figuren deuten auf gleiche oder analoge Elemente hin.

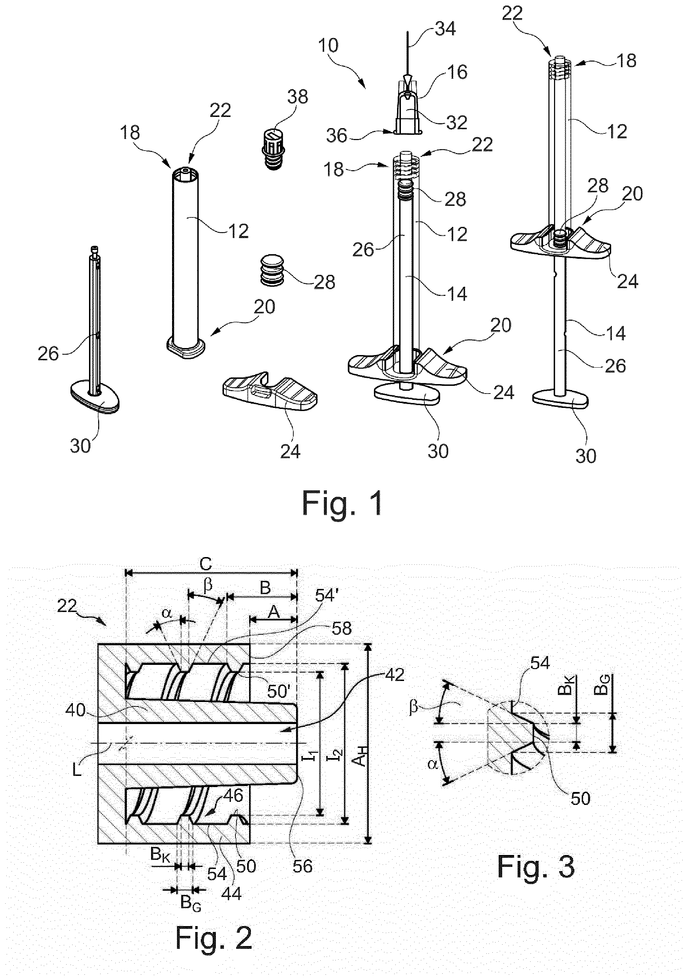

Figur 1 zeigt verschiedene perspektivische Ansichten einer Injektionsvorrichtung 10 gemäß einem Ausführungsbeispiel der Erfindung als Explosionsdarstellungen und im montierten Zustand. Die Injektionsvorrichtung 10 umfasst einen Spritzenkörper 12, eine Kolbenstangenanordnung 14 und eine Nadelanordnung 16.

Der längliche Spritzenkörper 12 ist hohlzylindrisch und bildet eine Kammer zum Aufnehmen eines Mediums, insbesondere eines hochviskosen Mediums. Der Spritzenkörper 12 hat einen distalen Endabschnitt 18 und einen proximalen Endabschnitt 20. Der proximale Endabschnitt 20 weist eine Öffnung auf, durch die die Kolbenstangenanordnung 14 in die Kammer einführbar ist (siehe Ansichten auf der rechten Seite in Fig. 1). An dem distalen Endabschnitt 18 des Spritzenkörpers 12 ist ein Luer-Lock-Verbinder 22 ausgebildet, welcher im Detail in Bezug auf Fig. 2 beschrieben ist. Der proximalen Endabschnitt 20 des Spritzenkörpers 12 ist mit einem Flansch 24 versehen, der als ein Griff zum Abstützen von Zeigefinder und Mittelfinger eines Anwenders bei der Verwendung des Spritzenkörpers 12 dient.

Die Kolbenstangenanordnung 14 umfasst eine Kolbenstange 26 und einen Kolben 28, der in dem Ausführungsbeispiel aus einem elastischen Material hergestellt ist und drei Dichtlippen aufweist. Der Kolben 28 ist an einem distalen Ende der Kolbenstange 26 angebracht und kann über eine Innenumfangsfläche der Kammer des Spritzenkörpers 12 in der Kammer gleitend geführt und in Richtung einer Kammerlängsachse verlagerbar sein. An einem proximalen Ende umfasst die Kolbenstangenanordnung 14 einen Griff 30, über den mittels eines Daumens des Anwenders Kraft auf die Injektionsvorrichtung 10 ausgeübt werden kann.

Die Nadelanordnung 16 umfasst einen Nadelansatz 32 und eine Kanüle 34 in Form einer Hohlnadel. In dem gezeigten Ausführungsbeispiel hat die Kanüle 34 eine Dicke von mindestens 30 G und eine Länge von 13 mm. Es können jedoch auch Kanülen mit anderen Maßen verwendet werden. Der Nadelansatz 32 weist ein Luer-Lock-Verbinder-Gegenstück 36 auf, welches mit dem Luer-Lock-Verbinder 22 des Spritzenkörpers 12 eine dichte Luer-Lock-Verbindung herstellen kann, um den Spritzenkörper 12 mit der Nadelanordnung 14 zu verbinden. Das Luer-Lock-Verbinder-Gegenstück 36, genauer gesagt der Nadelansatz 32, umfasst in dem gezeigten Ausführungsbeispiel einen Innenkegel und zwei an einer Außenumfangsfläche eines proximalen Endes des Nadelansatzes 32 ausgebildete Finnen. Es versteht sich, dass der Nadelansatz in weiteren Ausführungsformen mit einem Außengewinde bzw. einem Außengewindeabschnitt anstelle von Finnen versehen sein kann. Mittels einer solchen Ausführungsform mit Außengewinde kann eine noch sicherere Luer-Lock-Verbindung realisiert werden.

Um den Spritzenkörper 12 beispielsweise während des Transports zu verschließen, kann dieser im Bereich des Luer-Lock-Verbinders 22 mittels des in Figur 1 gezeigten Deckels 38 verschlossen sein. Dieser Deckel 38 ist komplementär zu dem Luer-Lock-Verbinder 22 des Spritzenkörpers 12 ausgebildet. Der Deckel 38 wird von dem Spritzenkörper 12 entfernt, bevor die Nadelanordnung 16 mit dem Spritzenkörper 12 verbunden wird.

Die Injektionsvorrichtung 10 kann in dem zusammengebauten Zustand, in dem der Spritzenkörper 12, die Kolbenstangenanordnung 14 und die Nadelanordnung 16 miteinander strukturell verbunden und wirkverbunden sind, aus einer Nichtbetätigungsstellung in eine Betätigungsstellung gebracht werden, indem der Kolben 28 innerhalb der Kammer von dem proximalen Endabschnitt 20 in Richtung des distalen Endabschnitts 18 verlagert wird. Hierdurch kann ein in der Kammer aufgenommenes hochviskoses Medium, beispielsweise Hyaluronsäure, durch die Kanüle 34 unter die Hautoberfläche eines Patienten gespritzt werden.

Der Spritzenkörper 12 ist in dem gezeigten Ausführungsbeispiel aus Cycloolefin-Polymer (COP) oder aus Cycloolefin-Copolymer (COC) hergestellt.

Figur 2 zeigt eine vergrößerte Schnittdarstellung des Luer-Lock-Verbinders 22 des Spritzenkörpers 12. Der Luer-Lock-Verbinder 22 umfasst einen Außenkegel 40 mit einer weiteren Öffnung 42 zum Ausgeben des in der Kammer aufgenommenen hochviskosen Mediums. Wie in den Figuren 1 und 2 zu erkennen ist, steht der Außenkegel 42 als eine konisch geformte Düse über ein distales Ende des Spritzenkörpers 12 hinaus. Der Luer-Lock-Verbinder 22 umfasst zudem einen hülsenförmigen Abschnitt 44, der mit einem Innengewinde 46 versehen ist. Der Außenkegel 40 ist von dem hülsenförmigen Abschnitt 44 umgeben, wobei der Außenkegel 40 und der hülsenförmige Abschnitt 44 koaxiale zueinander angeordnet sind.

Das Innengewinde 46 hat einen kleinsten Innendurchmesser I₁ von 7,1 mm, gemessen von Gewindekamm 50 zu gegenüberliegendem Gewindekamm 50' des Innengewindes 46. Der größte Innendurchmesser I₂, gemessen von Gewindegrund 54 zu gegenüberliegendem Gewindegrund 54' des Innengewindes 46, beträgt in dem gezeigten Ausführungsbeispiel 8,0 mm. Der Außendurchmesser A_{H} des hülsenförmigen Abschnitts 44 beträgt 10,0 mm. Demnach hat der hülsenförmige Abschnitt 44 eine Wandstärke von 1 mm.

Wie aus Figur 2 und insbesondere aus der Detailansicht des Gewindeprofils des Innengewindes 46 in Figur 3 zu erkennen ist, hat das Innengewinde 46 an dem Kamm 50 des Gewindeprofils eine Breite B_{K} von 0,48 mm. Ferner hat das Innengewinde 46 an dem Grund 54 des Gewindeprofils eine Breite B_{G} von 0,9 mm. Das Innengewinde weist an dem Gewindeprofil beidseitig einen Winkel α und β von jeweils 25° auf. Ein mit diesen Maßen ausgebildetes Gewindeprofil dient dazu, die gemeinsame Kontaktfläche mit dem komplementären Außengewinde der zugehörigen Nadelanordnung optimal festzulegen. Dies kann zur gezielten Einstellung der NPO-Mindestwiderstandsfähigkeit, der durchschnittlichen NPO-Widerstandsfähigkeit, Leakage-Mindestwiderstandsfähigkeit und der durchschnittlichen Leakage-Widerstandsfähigkeit beitragen.

Der Außenkegel 40 umfasst eine distale Stirnfläche 56, in der die weitere Öffnung 42 ausgebildet ist. Die distale Stirnfläche 56 steht in Richtung der Längsachse L des Spritzenkörpers 12 betrachtet um einen Abstand A von 2,3 mm über einen distalen Kragen 58 des hülsenförmigen Abschnitts 44 hinaus. Des Weiteren ist die distale Stirnfläche 56 des Außenkegels 40 von einer von der Stirnfläche 56 abgewandten Unterseite des ersten vollständigen Gewindeprofils um einen Abstand B von 3,1 mm beabstandet. Der Außenkegel 40 hat in dem gezeigten Ausführungsbeispiel eine Kegelgesamtlänge C von 8,9 mm.

Insbesondere die in Figur 3 gezeigten Parameter B_{K}, B_{G}, α und β des Gewindeprofils, der kleinste Innendurchmesser I₁ und/oder der Abstand A können mit diesen Maßen bei Spritzenkörpern mit verschiedenen Kammervolumina vorgesehen sein, um optimale Werte für die NPO-Mindestwiderstandsfähigkeit, für die durchschnittliche NPO-Widerstandsfähigkeit, für die Leakage-Mindestwiderstandsfähigkeit und für die durchschnittliche Leakage-Widerstandsfähigkeit des Spritzenkörpers 12 zu erreichen.

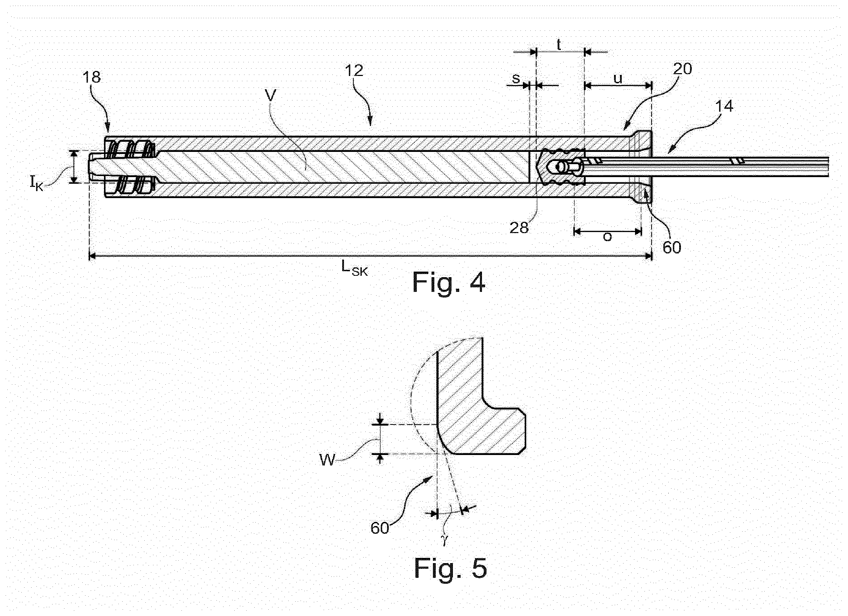

In Figur 4 ist eine Schnittdarstellung des Spritzenkörpers 12 mit einer darin aufgenommenen Kolbenstangenanordnung 14 gezeigt, wobei die Kolbenstangenanordnung 14 aus Übersichtsgründen nicht vollständig abgebildet ist. Der Spritzenkörper 12 des gezeigten Ausführungsbeispiels hat eine Gesamtlänge L_{SK} von 80,0 mm. Die Gesamtlänge L_{SK} setzt sich aus der Länge eines die Kammer bildenden Abschnitts des Spritzenkörpers und der Kegelgesamtlänge C des Außenkegels 40 zusammen. Der Kammerinnendurchmesser I_{K} der Kammer beträgt in dem gezeigten Beispiel 5 mm. Damit kann in der Kammer des Spritzenkörpers 12 und dem sich daran anschließenden durch den Außenkegel 40 gebildeten Kammerabschnitt zusammen ein Medium, insbesondere ein hochviskoses Medium, mit einem Volumen V von 1073 mm³ aufgenommen werden. Dieses aufnehmbare Volumen V ergibt sich aus den Überlegungen und resultierenden Gestaltungsmaßnahmen, wonach der Spritzenkörper 12 ein hochviskoses Medium von 1 ml Volumen bereitstellen soll. Zudem muss die Kammer ein Überfüllen von 2% und ein Einschließen einer Luftblase von 1 mm Länge (siehe Länge s) ermöglichen. Zusätzlich zu dem aufzunehmenden Volumen des Mediums samt vorstehend beschriebenen Toleranzen für das Überfüllen und die Luftblase, muss die Kammer bereits in einer Nichtbetätigungsstellung einen Teil der Kolbenstangenanordnung aufnehmen können und diese stabil lagern. Hierzu soll ein proximales Ende des Kolbens 28 der Kolbenstangenanordnung 14 in einer Nichtbetätigungsstellung bereits um einen Abstand u von 9,5 mm von dem proximalen Ende des Spritzenkörpers 12 entfernt beabstandet sein. Ferner ist eine dritte (proximale) Dichtlippe des Kolbens 28 der Kolbenstangenanordnung 14 in dieser Nichtbetätigungsstellung von einer Fase 60 des Spritzenkörpers 12 um einen Abstand o von ebenfalls 9,5 mm beabstandet. Der Kolben 28 hat beispielsweise eine Länge t von 6,9 mm.

Die Fase 60 ist an der Innenumfangsfläche des proximalen Endabschnitts 20 des Spritzenkörpers 12 ausgebildet. Die Fase 60 verjüngt die Innenumfangsfläche des Spritzenkörpers 12 von dem proximalen Endabschnitt 20 in Richtung des distalen Endabschnitts 18 betrachtet.

Die Fase 60 ist in Figur 5 vergrößert dargestellt. Sie hat in Richtung einer Längsachse L des Spritzenkörpers 12 betrachtet eine Länge w von 1,5 mm und schließt mit der Längsachse L einen Winkel γ von 15° ein. In einem Übergangsbereich der Fase 60 zu dem proximalen Ende des Spritzenkörpers 12 ist ein Radius R von 0,5 mm vorgesehen.

Der Spritzenkörper 12 des dargestellten Ausführungsbeispiels hat im Bereich der Kammer eine Wandstärke von 2,2 mm, was einer ausreichenden Stabilität gegen Bruch des Spritzenkörpers dient.

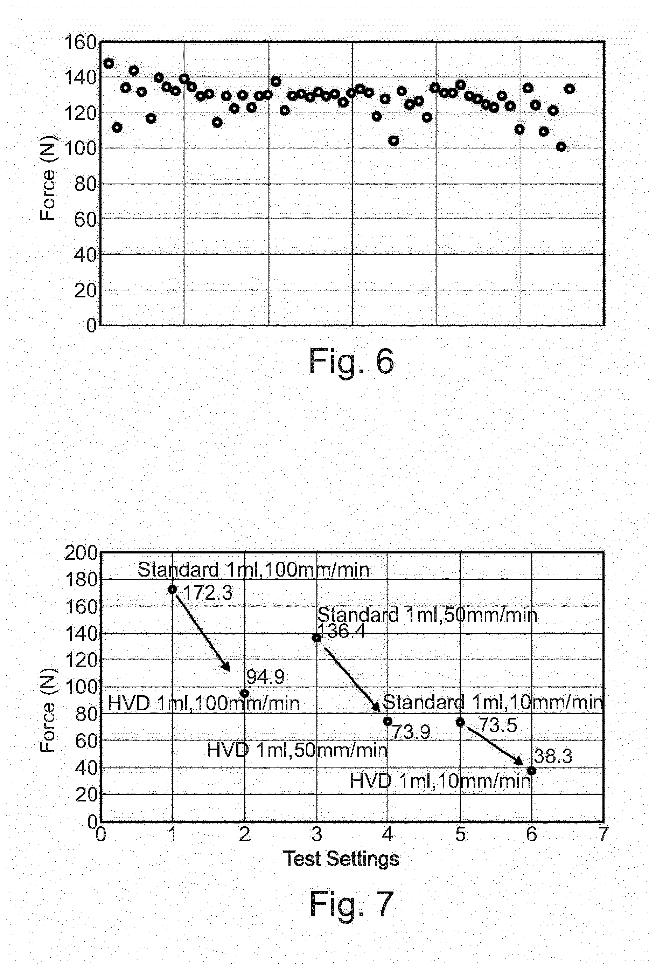

Figur 6 zeigt ein Diagramm mit Testergebnissen zu einem erfindungsgemäßen Spritzenkörper, die verschiedene gemessene Widerstandsfähigkeiten des Spritzenkörpers gegen zu NPO und Leakage zeigen. Die zugrundeliegenden Tests wurden mit dem Spritzenkörper 12 der Figuren 1 bis 5 durchgeführt. In Richtung der x-Achse des Diagramms sind die verschiedenen Messungen der dargestellten Messreihe (56 Einzelmessungen) aufgetragen. Die y-Achse gibt die gemessene Kraft an, die zum Zeitpunkt eines Versagens des Spritzenkörpers durch NPO oder Leakage auf die Spritze aufgebracht wurde.

Für das Testverfahren wurde der zu testende Spritzenkörper 12 senkrecht in der Universalprüfmaschine der Firma "TesT", Model "TesT 106.2 kN angeordnet und im Bereich des proximalen Endes des Spritzenkörpers 12 gehalten. Vor Testbeginn wurde der getestete Spritzenkörper 12 über den Luer-Lock-Verbinder 22 mit der Nadelanordnung "TSK STERiJECT Hypodermic Needle", Ref.: PRC-30013I, 30 G x ½ verbunden, die zum Herstellen der Luer-Lock-Verbindung als Luer-Lock-Verbinder-Gegenstück einen Innenkegel und zwei an einem Außenumfang eines Nadelansatzes der Nadelanordnung angeordnete Finnen umfasst. Der Luer-Lock-Verbinder 22 des Spritzenkörpers 12 wurde mit einem Drehmoment von 12 Ncm mit dem Luer-Lock-Verbinder-Gegenstück der Nadelanordnung verschraubt. Die Kanüle der Nadelanordnung, die eine Dicke von 30 G und eine Länge von 13 mm (30 G x ½) hat, wurde vor Durchführung des Testverfahrens mittels eines Hammers geplättet und dadurch verschlossen. Für das Testverfahren wurde eine trockene Luer-Lock-Verbindung hergestellt, indem die Nadelanordnung auf den Spritzenkörper aufgeschraubt wurde bevor anschließend ein hochviskoses Medium in die Kammer gefüllt wurde. Das Testverfahren wurde mit nicht dampfsterilisierten Komponenten (Spritzenkörper, Nadelanordnung, Kolbenstangenanordnung) durchgeführt. Für das vorliegende Testverfahren wurde ein hochviskoses Placebo-Medium verwendet. Das hochviskose Placebo-Medium hatte einen Speichermodul G' von etwa 84,5 Pa und einen Verlustfaktor tan δ von etwa 0,48. Die Viskosität wurde mit einem Platte-Platte-Messsystem bei 25°C und einem Luftdruck von 1013,25 hPa gemessen (z.B. Rheometer Firma: Anton Paar MCR 302). Die Frequenz betrug 1 Hz. Die Messung wurde nach ISO Norm 6721-10-2015-09 durchgeführt. Der getestete Spritzenkörper 12 wurde vollständig mit diesem Material befüllt, d.h. mit einem Volumen von 1073 mm³. Für die Kolbenanordnung wurde ein Standardkolben der Art FM257 des Herstellers Daetwyler verwendet.

Über einen Prüfstempel der Prüfmaschine wurde eine Kraft senkrecht auf das proximale Ende der Kolbenstangenanordnung aufgebracht. Der Prüfstempel wurde mit einer konstanten Prüfgeschwindigkeit von 12,6 mm/min in Richtung des distalen Endabschnitts des Spritzenkörpers bewegt, wobei die auf die Kolbenstangenanordnung einwirkende Kraft kontinuierlich erhöht wurde. Als Maximalkraft wurde eine Kraft von 420 N eingestellt, bei der der Test abgebrochen worden wäre. Die einwirkende Kraft wurde mittels eines Kraftsensors mit einer Abtastrate von 200 Hz detektiert. Der Prüfstempel wurde kontinuierlich weiterbewegt bis Undichtigkeit (*"Leakage*") und/oder NPO erkannt wurde, d.h. bis die gemessene Kraft plötzlich um wenigstens 30 % abfiel.

Die einwirkende Kraft zum Zeitpunkt des Auftretens von Undichtigkeit und/oder NPO wurde für jede Messung dokumentiert und in dem in Figur 6 gezeigten Diagramm eingetragen. Das Diagramm der Figur 6 zeigt die Ergebnisse von einer Messreihe mit 56 Messungen. Die in dem Diagramm dargestellten Ergebnisse zeigen, dass für keine der 56 Messungen ein Versagen des Spritzenkörpers durch NPO oder Leakage unter einer Kraft von 100 N aufgetreten ist. Das Durchführen einer Anzahl von 56 Messungen stellt sicher, dass eine NPO-Mindestwiderstandsfähigkeit bestimmt werden kann, die dadurch definiert ist, dass unterhalb dieses Schwellenwerts bei nicht mehr als 1,8% der getesteten Spritzenkörper ein "Needle Pop Off" auftritt.

Da die maximale Fingerkraft, mit der Spritzen in der Praxis betätigt werden, bei durchschnittlich 95 N liegen, kann das Auftreten von NPO und/oder Leakage mit dem erfindungsgemäßen Spritzenkörper vermieden werden.

In Figur 7 ist ein weiteres Diagramm mit Testergebnissen zu Extrusionskräften eines erfindungsgemäßen Spritzenkörpers im Vergleich zu Extrusionskräften eines bekannten Produktes aus dem Stand der Technik. In Richtung der x-Achse des Diagramms sind die verschiedenen Messungen aufgetragen. Die y-Achse gibt die gemessene Extrusionskraft an. In dem Diagramm sind Messungen mit drei verschiedenen Prüfgeschwindigkeiten gezeigt.

Verglichen wurde ein Spritzenkörper gemäß dem in den Figuren 1 bis 5 gezeigten Ausführungsbeispiel mit einem "SCHOTT COC Standard"-Spritzenkörper mit 1 ml Füllvolumen (Bezeichnung "TopPac 1 ml long"). Für die Kolbenanordnung wurde mit beiden Spritzenkörpern ein Standardkolben der Art FM257 des Herstellers Daetwyler verwendet. Als Nadelanordnung wurde mit beiden Spritzenkörpern die Nadelanordnung "TSK HYPODERMIC NEEDLE", Ref.: HPC-30013I-320, 30 G x ½ mit einer offenen (nicht geplätteten) Kanüle mit einer Dicke von 30 G und einer Länge von 13 mm verwendet. Als extrudiertes Medium wurde ein hochviskoses Medium verwendet. Das hochviskose Medium hatte einen Speichermodul G' von etwa 84,5 Pa und einen Verlustfaktor tan δ von etwa 0,48. Die Viskosität wurde mit einem Platte-Platte-Messsystem bei 25°C und einem Luftdruck von 1013,25 hPa gemessen (z.B. Rheometer Firma: Anton Paar MCR 302). Die Frequenz betrug 1 Hz. Die Messung wurde nach ISO Norm 6721-10-2015-09 durchgeführt.

Geprüft wurde mit Prüfgeschwindigkeiten von 100 mm/min, 50 mm/min und 10 mm/min, mit denen der Kolben in dem Spritzenkörper von proximal nach distal verlagert wurde. Die Testergebnisse zeigen, dass bei einer Prüfgeschwindigkeit von 100 mm/min die Extrusionskraft des erfindungsgemäßen Spritzenkörpers gegenüber dem Spritzenkörper aus dem Stand der Technik von 172,3 N auf 94,9 N verringert wurde. Ferner zeigen die Testergebnisse, dass bei einer Prüfgeschwindigkeit von 50 mm/min die Extrusionskraft des erfindungsgemäßen Spritzenkörpers gegenüber dem Spritzenkörper aus dem Stand der Technik von 136,4 N auf 73,9 N verringert wurde. Des Weiteren zeigen die Testergebnisse, dass bei einer Prüfgeschwindigkeit von 10 mm/min die Extrusionskraft des erfindungsgemäßen Spritzenkörpers gegenüber dem Spritzenkörper aus dem Stand der Technik von 73,5 N auf 38,3 N verringert wurde.

Die Extrusionskräfte des erfindungsgemäßen Spritzenkörper 12 sind somit signifikant geringer als die Extrusionskräfte bekannter Spritzenkörper. Der erfindungsgemäße Spritzenkörper erfordert somit weniger Kraftaufbringung durch den Anwender zum Ausgeben hochviskoser Medien und ermöglicht dadurch eine verbesserte Dosierungsgenauigkeit. Somit erlauben die erfindungsgemäßen Spritzenkörper trotz der Verwendung hochviskoser Medien eine Nutzung von Nadelanordnungen mit Kanülen geringer Dicke, d.h. einer Dicke von 30 G oder mehr.

### Bezugszeichenliste

| | | | |
|---|---|---|---|
| 10 | Injektionsvorrichtung | A | Abstand |
| 12 | Spritzenkörper | B | Abstand |
| 14 | Kolbenstangenanordnung | C | Kegelgesamtlänge |
| 16 | Nadelanordnung | I₁ | kleinster Innendurchmesser |
| 18 | distaler Endabschnitt | I₂ | größter Innendurchmesser |
| 20 | proximaler Endabschnitt | A_{H} | Außendurchmesser |
| 22 | Luer-Lock-Verbinder | B_{K} | Breite Kamm |
| 24 | Flansch | B_{G} | Breite Grund |
| 26 | Kolbenstange | α | Winkel am Gewindeprofil |
| 28 | Kolben | β | weiterer Winkel am Gewindeprofil |
| 30 | Griff | Y | Winkel der Fase |
| 32 | Nadelansatz | I_{K} | Kammerinnendurchmesser |
| 34 | Kanüle | V | aufnehmbares Volumen |
| 36 | Finne | L_{SK} | Gesamtlänge |
| 38 | Deckel | o | Abstand |
| 40 | Außenkegel | s | Länge der Luftblase |
| 42 | Öffnung | t | Kolbenlänge |
| 44 | hülsenförmiger Abschnitt | u | Abstand |
| 46 | Innengewinde | w | Länge der Fase |
| 50, 50' | Kamm | | |
| 54, 54' | Grund | | |
| 56 | Stirnfläche | | |
| 58 | distaler Kragen | | |
| 60 | Fase | | |
| L | Längsachse | | |

## Patentansprüche

1. Injektionsvorrichtung (10) zum Injizieren eines hochviskosen Mediums, umfassend eine Spritze zum Injizieren eines hochviskosen Mediums mit einem Spritzenkörper (12) und einer Kolbenstangenanordnung (14) und eine Nadelanordnung (16) mit einem Nadelansatz (32) und einer Kanüle (34),
wobei der Spritzenkörper (12) einen distalen Endabschnitt (18) und einen proximalen Endabschnitt (20) umfasst,
wobei der Spritzenkörper (12) hohlzylindrisch ist und eine Kammer zum Aufnehmen des hochviskosen Mediums bildet,
wobei der proximale Endabschnitt (20) eine Öffnung aufweist, durch die eine Kolbenstangenanordnung (14) in die Kammer einführbar ist,
wobei die Kolbenstangenanordnung (14) eine Kolbenstange (26) und einen an ein distales Ende der Kolbenstange (26) angebrachten Kolben (28) umfasst, wobei der Kolben (28) über die Öffnung an dem proximalen Endabschnitt (20) des Spritzenkörpers (12) in der Kammer aufgenommen und in dieser verlagerbar geführt ist,
wobei an dem distalen Endabschnitt (18) des Spritzenkörpers (12) ein Luer-Lock-Verbinder (22) ausgebildet ist, der einen Außenkegel (40) mit einer weiteren Öffnung (42) zum Ausgeben des hochviskosen Mediums und einen hülsenförmigen Abschnitt (44) mit einem Innengewinde (46) umfasst,
wobei der Nadelansatz (32) zum Herstellen einer Luer-Lock-Verbindung zwischen dem Spritzenkörper (12) und der Nadelanordnung (16) ein zu dem Luer-Lock-Verbinder (22) des Spritzenkörpers (12) komplementäres Luer-Lock-Verbinder-Gegenstück (36) umfasst,
und wobei der Spritzenkörper (12) für eine Luer-Lock-Verbindung des Luer-Lock-Verbinders (22) mit dem Luer-Lock-Verbinder-Gegenstück (36) eine NPO-Mindestwiderstandsfähigkeit - gemessen nach dem in der Beschreibung auf Seite 3 bis Seite 5 spezifizierten Verfahren - zwischen 90 N und 105 N hat,
wobei der Spritzenkörper an dem proximalen Endabschnitt (20) eine Innenumfangsfläche mit einer Fase (60) umfasst, die die Innenumfangsfläche von dem proximalen Endabschnitt (20) in Richtung des distalen Endabschnitts (18) betrachtet verjüngt, **dadurch gekennzeichnet dass**, die Fase (60) in Richtung einer Längsachse (L) des Spritzenkörpers (12) betrachtet eine Länge (w) zwischen 1,2 mm und 1,8 mm, vorzugsweise von 1,4 mm bis 1,6 mm, bevorzugt von 1,5 mm hat und/oder wobei die Fase (60) einen Winkel (γ) von wenigstens 13°, vorzugsweise von wenigstens 14°, bevorzugt von wenigstens 15° mit der Längsachse (L) einschließt.

2. Injektionsvorrichtung (10) nach Anspruch 1, wobei der Spritzenkörper (12) eine durchschnittliche NPO-Widerstandsfähigkeit von wenigstens 110 N, bevorzugt wenigstens 115 N, weiter bevorzugt wenigstens 117 N hat.

3. Injektionsvorrichtung (10) nach Anspruch 1 oder 2, wobei das Innengewinde (46) einen kleinsten Innendurchmesser (I₁) zwischen 7,05 mm und 7,15 mm, vorzugsweise zwischen 7,08 mm und 7,12 mm, bevorzugt von 7,1 mm hat.

4. Injektionsvorrichtung (10) nach einem der vorangehenden Ansprüche, wobei das Innengewinde (46) an dem Kamm (50) des Gewindeprofils eine Breite (B_{K}) zwischen 0,44 mm und 0,52 mm, vorzugsweise zwischen 0,46 mm und 0,50 mm, bevorzugt von 0,48 mm hat.

5. Injektionsvorrichtung (10) nach einem der vorangehenden Ansprüche, wobei das Innengewinde (46) an dem Grund (54) des Gewindeprofils eine Breite (B_{G}) zwischen 0,85 mm und 0,95 mm, vorzugsweise zwischen 0,875 mm und 0,925 mm, bevorzugt von 0,9 mm hat.

6. Injektionsvorrichtung (10) nach einem der vorangehenden Ansprüche, wobei eine distale Stirnfläche (56) des Außenkegels (40) um einen Abstand (A) von 2,1 mm bis 2,5 mm, vorzugsweise von 2,2 mm bis 2,4 mm, bevorzugt von 2,3 mm über einen distalen Kragen (58) des hülsenförmigen Abschnitts (44) hinaussteht.

7. Injektionsvorrichtung (10) nach einem der vorangehenden Ansprüche, wobei der Spritzenkörper eine maximale Gesamtlänge (L_{SK}) von 80 mm und einen Kammerinnendurchmesser (I_{K}) von maximal 5 mm hat, wobei in dem Spritzenkörper (12) ein Volumen (V) von wenigstens 1 ml aufnehmbar ist.

8. Injektionsvorrichtung (10) nach einem der vorangehenden Ansprüche, wobei der Spritzenkörper zumindest im Bereich der Kammer eine Wandstärke von wenigstens 1,7 mm, vorzugsweise wenigstens 1,8 mm, bevorzugt wenigstens 2,0 mm, weiter bevorzugt wenigstens 2,2 mm aufweist.

9. Injektionsvorrichtung (10) nach einem der vorangehenden Ansprüche, wobei der Spritzenkörper aus einem Kunststoffmaterial hergestellt ist, das vorzugsweise einen E-Modul zwischen 2800 MPa und 3300 MPa hat, insbesondere zwischen 2900 MPa und 3200 MPa.

10. Injektionsvorrichtung (10) nach einem der vorangehenden Ansprüche, wobei
die Kanüle (34) eine Dicke von mindestens 31 G, vorzugsweise mindestens 32 G, bevorzugt von mindestens 33 G, weiter bevorzugt von mindestens 34 G, noch weiter bevorzugt von mindestens 35 G hat und/oder wobei
der Nadelansatz (32) einen Innenkegel umfasst, der entlang einer Längsachse der Nadelanordnung (16) eine Länge zwischen 3 mm und 7 mm, vorzugsweise zwischen 5,5 mm und 6,5 mm, bevorzugt von 6.1 mm hat.

11. Kosmetisches Verfahren, umfassend
Applizieren einer hochviskosen kosmetischen Zubereitung unter Verwendung einer Injektionsvorrichtung nach einem der Ansprüche 1 bis 10.

## Claims

1. Injection device (10) for injecting a highly viscous medium, comprising a syringe for injecting a highly viscous medium having a syringe body (12) and a piston rod assembly (14) and a needle assembly (16) comprising a needle hub (32) and a cannula (34),
wherein the syringe body (12) comprises a distal end portion (18) and a proximal end portion (20),
wherein the syringe body (12) is hollow cylindrical and forms a chamber for receiving the highly viscous medium,
wherein the proximal end portion (20) comprises an opening through which a piston rod assembly (14) is insertable into the chamber,
wherein the piston rod assembly (14) comprises a piston rod (26) and a piston (28) attached to a distal end of the piston rod (26), wherein the piston (28) is received in and displaceably guided within said chamber via said opening at the proximal end portion (20) of the syringe body (12),
wherein a Luer lock connector (22) is formed at the distal end portion (18) of the syringe body (12), which connector (22) comprises an outer cone (40) having a further opening (42) for dispensing the highly viscous medium and a sleeve-shaped portion (44) comprising an internal thread (46),
wherein the needle hub (32) comprises a Luer lock connector counterpart (36) complementary to the Luer lock connector (22) of the syringe body (12) for establishing a Luer lock connection between the syringe body (12) and the needle assembly (16),
and wherein for a Luer lock connection of the Luer lock connector (36) with the Luer lock connector counterpart (36) the syringe body (12) has a minimum NPO resistance between 90 N and 105 N, as measured according to the method specified in the description on pages 3 to 5,
wherein the syringe body at the proximal end portion (20) comprises an inner peripheral surface having a chamfer (60) which causes the inner peripheral surface to taper as viewed from the proximal end portion (20) in the direction of the distal end portion (18),
**characterized in that**,
the chamfer (60) as viewed in the direction of a longitudinal axis (L) of the syringe body (12) has a length (w) between 1.2 mm and 1.8 mm, preferably from 1.4 mm to 1.6 mm, more preferably of 1.5 mm, and/or wherein the chamfer (60) forms an angle (y) of at least 13°, preferably at least 14°, more preferably at least 15°, with the longitudinal axis (L).

2. Injection device (10) according to claim 1, wherein the syringe body (12) has an average NPO resistance of at least 110 N, preferably at least 115 N, more preferably at least 117 N.

3. Injection device (10) according to claim 1 or 2, wherein the internal thread (46) has a smallest internal diameter (I₁) between 7.05 mm and 7.15 mm, preferably between 7.08 mm and 7.12 mm, more preferably 7.1 mm.

4. Injection device (10) according to any one of the preceding claims, wherein the internal thread (46) has a width (B_{K}) between 0.44 mm and 0.52 mm, preferably between 0.46 mm and 0.50 mm, more preferably 0.48 mm at the ridge (50) of the thread profile.

5. Injection device (10) according to any one of the preceding claims, wherein the internal thread (46) has a width (B_{G}) between 0.85 mm and 0.95 mm, preferably between 0.875 mm and 0.925 mm, more preferably 0.9 mm at the root (54) of the thread profile.

6. Injection device (10) according to any one of the preceding claims, wherein a distal end face (56) of the outer cone (40) projects by a distance (A) of 2.1 mm to 2.5 mm, preferably from 2.2 mm to 2.4 mm, more preferably from 2.3 mm, beyond a distal collar (58) of the sleeve-shaped portion (44).

7. Injection device (10) according to any one of the preceding claims, wherein the syringe body has a maximum overall length (L_{SK}) of 80 mm and a chamber inner diameter (I_{K}) of at most 5 mm, wherein a volume (V) of at least 1 ml can be received in the syringe body (12).

8. Injection device (10) according to any one of the preceding claims, wherein the syringe body has a wall thickness of at least 1.7 mm, preferably at least 1.8 mm, more preferably at least 2.0 mm, further preferably at least 2.2 mm, at least in the region of the chamber.

9. Injection device (10) according to any one of the preceding claims, wherein the syringe body is made of a plastic material which preferably has a modulus of elasticity between 2800 MPa and 3300 MPa, in particular between 2900 MPa and 3200 MPa.

10. Injection device (10) according to any one of the preceding claims, wherein the cannula (34) has a thickness of at least 31 G, preferably at least 32 G, more preferably at least 33 G, still more preferably at least 34 G, further preferably at least 35 G, and/or
the needle hub (32) comprises an inner cone having a length along a longitudinal axis of the needle assembly (16) between 3 mm and 7 mm, preferably between 5.5 mm and 6.5 mm, more preferably 6.1 mm.

11. Cosmetic method comprising
applying a highly viscous cosmetic preparation by use of an injection device according to any one of claims 1 to 10.

## Revendications

1. Dispositif d'injection (10) pour injecter un milieu hautement visqueux, comprenant une seringue pour injecter un milieu hautement visqueux, comprenant un corps de seringue (12) et un ensemble de tige de piston (14), et un ensemble d'aiguille (16) comprenant une embase d'aiguille (32) et une canule (34),
dans lequel le corps de seringue (12) comprend une partie d'extrémité distale (18) et une partie d'extrémité proximale (20),
dans lequel le corps de seringue (12) est cylindrique creux et forme une chambre pour recevoir le milieu hautement visqueux,
dans lequel la partie d'extrémité proximale (20) comporte une ouverture à travers laquelle un ensemble de tige de piston (14) peut être inséré dans la chambre,
dans lequel l'ensemble de tige de piston (14) comprend une tige de piston (26) et un piston (28) monté sur une extrémité distale de la tige de piston (26), le piston (28) étant reçu dans la chambre par l'intermédiaire de l'ouverture sur la partie d'extrémité proximale (20) du corps de seringue (12) et étant guidé de manière déplaçable dans la chambre,
dans lequel un connecteur Luer-Lock (22) est formé sur la partie d'extrémité distale (18) du corps de seringue (12), lequel comprend un cône extérieur (40) avec une autre ouverture (42) pour distribuer le milieu très visqueux et une partie en forme de manchon (44) avec un filetage intérieur (46),
dans lequel l'embase d'aiguille (32) destiné à établir une connexion Luer-Lock entre le corps de seringue (12) et l'ensemble d'aiguille (16) comprend une contre-pièce de connecteur Luer-Lock (36) complémentaire du connecteur Luer-Lock (22) du corps de seringue (12),
et dans lequel le corps de seringue (12) a une résistance NPO minimale, mesurée selon la méthode spécifiée dans la description de la page 3 à la page 5, comprise entre 90 N et 105 N pour une connexion Luer-Lock du connecteur Luer-Lock (22) avec le connecteur Luer-Lock complémentaire (36),
dans lequel le corps de seringue comprend, au niveau de la partie d'extrémité proximale (20), une surface périphérique intérieure avec un chanfrein (60) qui rétrécit la surface périphérique intérieure lorsqu'elle est vue depuis la partie d'extrémité proximale (20) en direction de la partie d'extrémité distale (18), **caractérisé en ce que**, le chanfrein (60), vu dans la direction d'un axe longitudinal (L) du corps de seringue (12), a une longueur (w) comprise entre 1,2 mm et 1,8 mm, de préférence entre 1,4 mm et 1,6 mm, de préférence de 1,5 mm et/ou dans lequel le chanfrein (60) forme un angle (y) d'au moins 13°, de préférence d'au moins 14°, de préférence d'au moins 15° avec l'axe longitudinal (L).

2. Dispositif d'injection (10) selon la revendication 1, dans lequel le corps de seringue (12) a une résistance NPO moyenne d'au moins 110 N, de préférence d'au moins 115 N, de préférence encore d'au moins 117 N.

3. Dispositif d'injection (10) selon la revendication 1 ou 2, dans lequel le filetage interne (46) présente un plus petit diamètre interne (I₁) compris entre 7,05 mm et 7,15 mm, de préférence entre 7,08 mm et 7,12 mm, de préférence de 7,1 mm.

4. Dispositif d'injection (10) selon l'une quelconque des revendications précédentes, dans lequel le filetage interne (46) au niveau de la crête (50) du profil de filetage a une largeur (Bₖ) comprise entre 0,44 mm et 0,52 mm, de préférence entre 0,46 mm et 0,50 mm, de préférence de 0,48 mm.

5. Dispositif d'injection (10) selon l'une quelconque des revendications précédentes, dans lequel le filetage interne (46) à la base (54) du profilé fileté présente une largeur (B_{G}) comprise entre 0,85 mm et 0,95 mm, de préférence entre 0,875 mm et 0,925 mm, de préférence de 0,9 mm.

6. Dispositif d'injection (10) selon l'une quelconque des revendications précédentes, dans lequel une face d'extrémité distale (56) du cône extérieur (40) dépasse d'un collet distal (58) de la partie en forme de manchon (44) d'une distance (A) de 2,1 mm à 2,5 mm, de préférence de 2,2 mm à 2,4 mm, de préférence de 2,3 mm.

7. Dispositif d'injection (10) selon l'une des revendications précédentes, dans lequel le corps de la seringue a une longueur totale maximale (L_{SK}) de 80 mm et un diamètre intérieur de chambre (I_{K}) de 5 mm au maximum, un volume (V) d'au moins 1 ml pouvant être logé dans le corps de la seringue (12).

8. Dispositif d'injection (10) selon l'une des revendications précédentes, dans lequel le corps de la seringue présente, au moins dans la zone de la chambre, une épaisseur de paroi d'au moins 1,7 mm, de préférence d'au moins 1,8 mm, de préférence d'au moins 2,0 mm, de préférence encore d'au moins 2,2 mm.

9. Dispositif d'injection (10) selon l'une des revendications précédentes, dans lequel le corps de la seringue est fabriqué en une matière plastique qui a de préférence un module d'élasticité compris entre 2800 MPa et 3300 MPa, en particulier entre 2900 MPa et 3200 MPa.

10. Dispositif d'injection (10) selon l'une quelconque des revendications précédentes, dans lequel la canule (34) a une épaisseur d'au moins 31 G, de préférence d'au moins 32 G, de préférence d'au moins 33 G, plus préférentiellement d'au moins 34 G, encore plus préférentiellement d'au moins 35 G et/ou dans lequel l'embase d'aiguille (32) comprend un cône interne qui, le long d'un axe longitudinal de l'ensemble d'aiguille (16), a une longueur comprise entre 3 mm et 7 mm, de préférence entre 5,5 mm et 6,5 mm, de préférence de 6,1 mm

11. Procédé cosmétique comprenant
Appliquer une préparation cosmétique à haute viscosité en utilisant un dispositif d'injection selon l'une quelconque des revendications 1 à 10.
